(19) Europäisches Patentamt
European Patent Office
Office européen des brevets

(11) Publication number: **0 499 179 A1**

# (12) EUROPEAN PATENT APPLICATION

(21) Application number: **92102199.4**

(22) Date of filing: **10.02.92**

(51) Int. Cl.5: **C07D 487/04**, //A61K31/40, (C07D487/04,209:00,209:00)

(30) Priority: **14.02.91 IT 38891**

(43) Date of publication of application: **19.08.92 Bulletin 92/34**

(84) Designated Contracting States: **PT**

(71) Applicant: **LABORATORIO CHIMICO INTERNAZIONALE S.p.A.**
**Via Salvini, 10**
**I-20122 Milano(IT)**

(72) Inventor: **Maiorana, Stefano**
**Via Salvini, 10**
**I-20122 Milano(IT)**
Inventor: **Bonura, Anna**
**Via Salvini, 10**
**I-20122 Milano(IT)**
Inventor: **Chiodini, Giorgio**
**Via Salvini, 10**
**I-20122 Milano(IT)**

(74) Representative: **Bianchetti, Giuseppe**
**Studio Consulenza Brevettuale Via Rossini, 8**
**I-20122 Milan(IT)**

## (54) A process for the preparation of 1,3a,8-trimethyl-1,2,3,3a,8,8a-hexahydro-pyrrole/2,3-b/indol-5(3aS,8aR)-heptylcarbamate.

(57) A process for the preparation of 1,3a,8-trimethyl-1,2,3,3a,8,8a-hexahydro-pyrrole[2,3-b]indol-5(3aS,8aR)-heptylcarbamate, of formula (I)

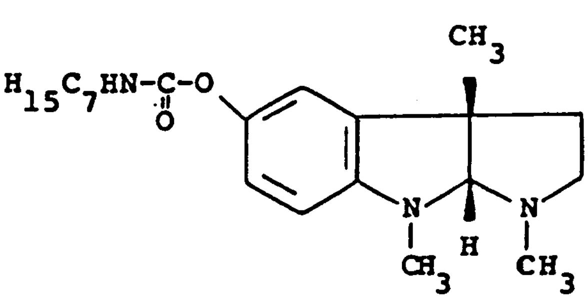

(I)

by reacting eseroline with heptylisocyanate in the presence of a metal cyanate and of a quaternary ammonium salt, in polar aprotic solvents.

EP 0 499 179 A1

The present invention relates to a process for the preparation of 1,3a,8-trimethyl-1,2,3,3a,8,8a-hexahydropyrrole[2,3-b]indol-5(3aS,8aR)-heptylcarbamate of formula (I):

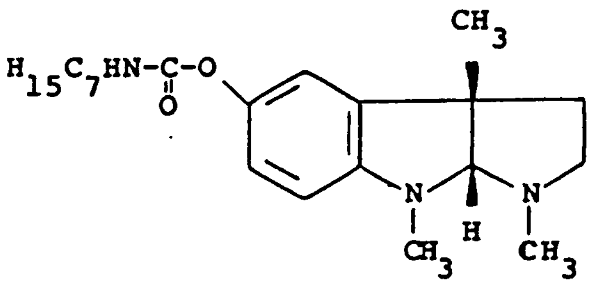

(I)

Said compound, which is known under the International Nonproprietary Name "heptastigmine", and methods for the preparation thereof, are disclosed in Italian Patent Applications N. 47780 A/84 and 19964 A/87. The compound has interesting pharmacological properties making it useful particularly in the treatment of Alzheimer's disease.

According to the above cited Patent Applications, heptastigmine (I) can be obtained starting from eseroline (II)

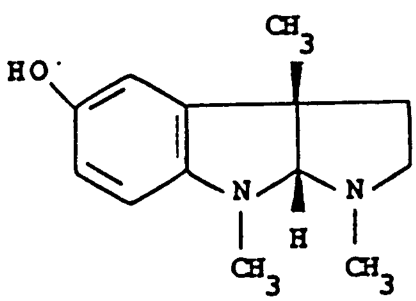

(II)

by treatment either with phosgene and heptylamine, or with heptylisocyanate. The latter method allows to obtain higher yields than the first one, as far as the reaction is carried out in an ether solution and in the presence of metal sodium.

Said methods, however, suffer form evident drawbacks, particularly on industrial scale. In fact, the first method involves the use of highly toxic phosgene, whereas the second one suffers from hazards of fires and explosions, connected to the use of ether and metal sodium.

Now, it has surprisingly been found that heptastigimine (I) can be obtained in very good yields and high purity, by reacting eseroline (II) with heptylisocyanate in the presence of a) catalytic amounts of a quaternary ammonium salt and b) catalytic amounts of a metal cyanate, in a polar aprotic solvent, according to the scheme reported hereinbelow:

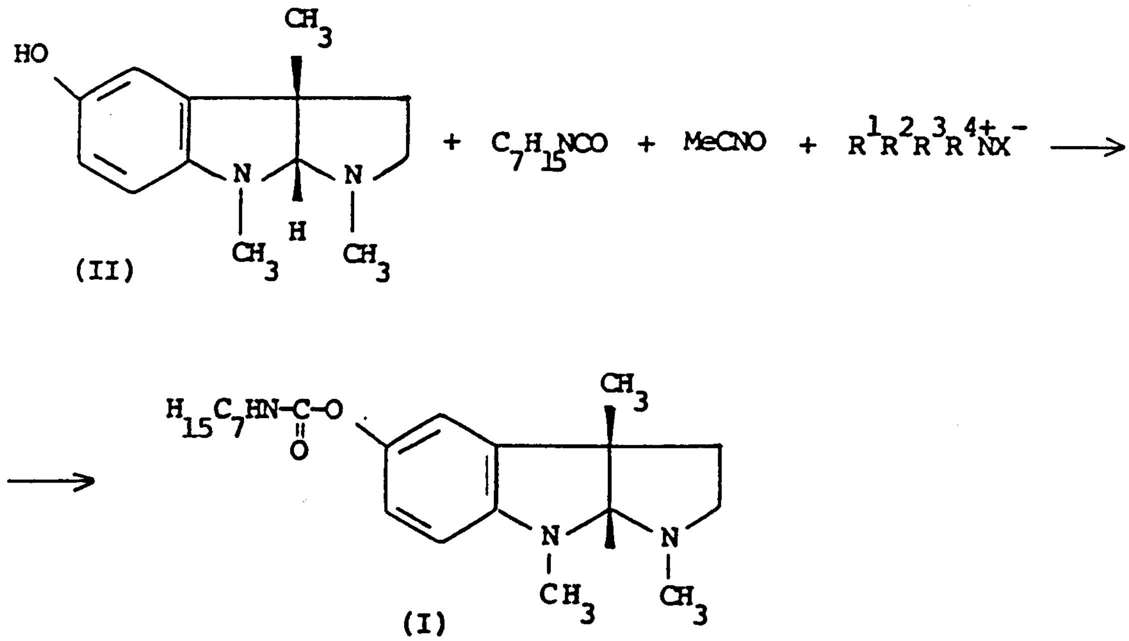


(II)

(I)

wherein Me is an alkali metal or the equivalent of an alkaline-earth metal, and preferably potassium; $R_1$-$R_4$, which can be the same or different, are $C_1$-$C_{12}$ alkyl or aralkyl, preferably ethyl or n-butyl; X is an anion, preferably chlorine or bromine.

The molar ratio of eseroline to heptylisocyanate is comprised between 1:1 and 1:5, preferably between 1:1 and 1:1.2. Heptylisocyanate amounts which are in excess or defect with respect to the preferred range can also be used, but no advantages are obtained.

The molar ratio of eseroline to metal cyanate can range between 1:0.5 and 1:0.01, preferably between 1:0.4 and 1:0.1, whereas the molar ratio of eseroline to quaternary ammonium salt is comprised between 1:0.6 and 1:0.05, preferably between 1:0.5 and 1:0.2.

Acetonitrile and ethyl acetate are preferably used as the polar aprotic solvent.

It has also been found that metal alcoholates, particularly potassium t-butylate, may substitute in some cases the metal cyanate.

The reaction is preferably carried out under nitrogen, in order to prevent eseroline oxidation, and at temperatures comprised between 0°C and 40°C, preferably at about 20°C. The order in which reagents are added is not particularly critic; however heptylisocyanate, optionally diluted with the solvent, is preferably added to a solution or suspension of eseroline, metal cyanate and quaternary ammonium salt in the solvent itself. At the end of the reaction (the progress of which can be followed with chromatographic methods, for example by TLC mix, eluent $CH_2Cl_2$/MeOH 9:1) solvent is evaporated under reduced pressure, the residue is taken up with toluene, the unsolubles are filtered off and the toluene solution is repeatedly washed with water. Toluene is evaporated off to obtain the desired product as a viscous oil, which is left to crystallize or is transformed into the corresponding tartrate, according to indications of Italian Patent Application N. 19964 A/87.

According to an alternative route, eseroline can be N-heptylcarbamoylated with heptylisocyanate which is prepared in situ starting from a 1-haloheptane (preferably 1-bromoheptane) and a metal cyanate (preferably potassium cyanate) in the presence of a quaternary ammonium salt, in polar aprotic solvents, preferably acetonitrile. In this case the reaction is carried out (always under nitrogen protection) at the reflux temperature of the mixture; compound (I) is then recovered as described above.

The following Examples further illustrate the invention.

## EXAMPLE 1

500 g (2.3 moles) of eseroline are dissolved in 20 l of anhydrous acetonitrile, under nitrogen; 37 g (0.46 mole) of potassium cyanate and 148 g (0.69 mole) of tetraethylammonium bromide are added thereto and, at 19-20°C, a solution of 325 g (2.5 moles) of heptylisocyanate in 20 l of anhydrous acetonitrile is quickly dropped therein. The reaction mixture is kept under stirring for 8 hours, then the insolubles are filtered off,

solvent is evaporated off under reduced pressure, the residue is taken up into 100 l of toluene and the toluene solution is washed with 6 x 100 l of water.

Toluene is evaporated under vacuum to yield a yellowish viscous oil which undergoes solidification and can be transformed into the corresponding tartrate according to the indications of Italian Patent Application n. 19964 A/87. Yield 90%.

**EXAMPLE 2**

150 g of eseroline (0.69 mole), 83.9 g of potassium cyanate (1.035 moles), 148 g of 1-bromoheptane (0.83 mole) and 29 g of tetraethylammonium bromide (0.14 mole) are placed into 5 l of anhydrous acetonitrile. The reaction mixture is refluxed for 19 hours, the insolubles are filtered off and the mixture is worked up as described in Example 1. Heptastigmine yield is 63%.

**EXAMPLE 3**

The procedure of Example 1 is followed, but replacing tetraethylammonium bromide with an equivalent amount of tetra-n-butylammonium bromide. The yield is still 90%.

**EXAMPLE 4**

The same results as those of Example 2 are obtained using n.$Bu_4NBr^-$ instead of $(C_2H_5)_4NBr^-$, the other reaction conditions being unchanged.

**Claims**

**1.** A process for the preparation of 1,3a,8-trimethyl-1,2,3,3a,8,8a-hexahydro-pyrrole[2,3-b]indol-5(3aS,8aR)-heptylcarbamate of formula (I):

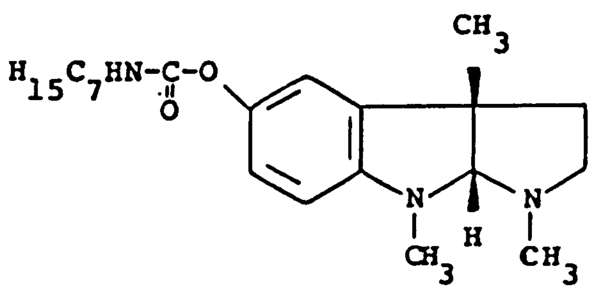

(I)

characterized in that eseroline (II) is reacted with heptylisocyanate in the presence of a) catalytic amounts of a quaternary ammonium salt and b) catalytic amounts of a metal cyanate, in a polar aprotic solvent, according to the scheme reported hereinbelow:

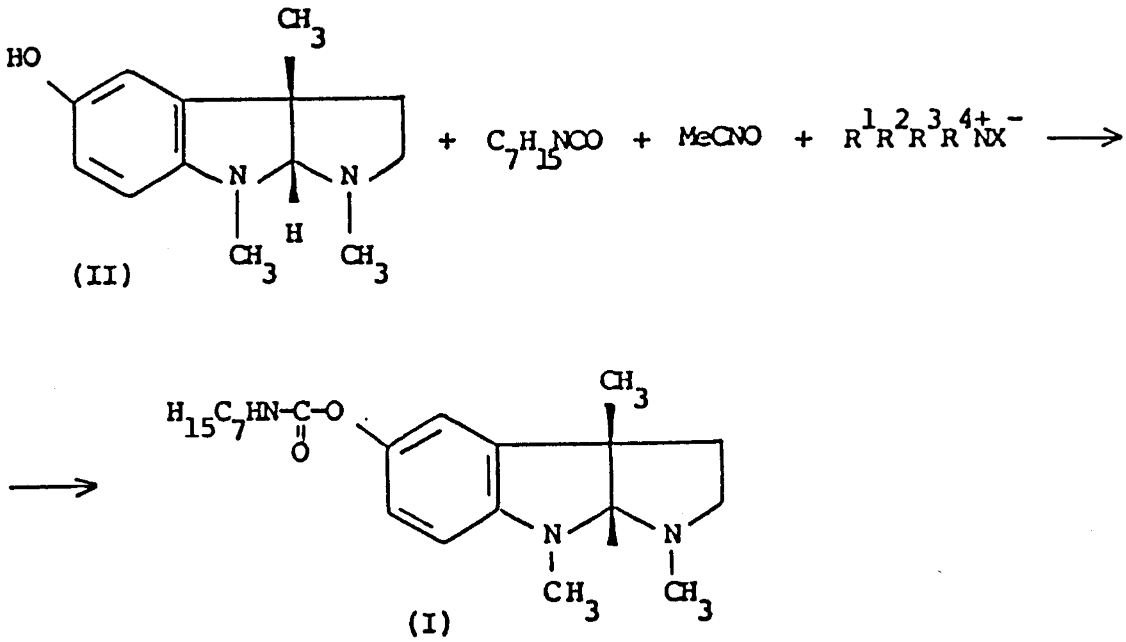

(II)

(I)

wherein Me is an alkali metal or the equivalent of an alkaline-earth metal; $R_1$-$R_4$, which can be the same or different, are $C_1$-$C_{12}$ alkyl or aralkyl; X is an anion.

**2.** A process according to claim 1, characterized in that the molar ratio of eseroline to heptylisocyanate is comprised between 1:1 and 1:5, preferably between 1:1 and 1:1.2.

**3.** A process according to claims 1 or 2, characterized in that the molar ratio of eseroline to metal cyanate is comprised between 1:0.5 and 1:0.01, preferably between 1:0.4 and 1:0.1.

**4.** A process according to any one of the previous claims, characterized in that the molar ratio of eseroline to quaternary ammonium salt is comprised between 1:0.6 and 1:0.05, preferably between 1:0.5 and 1:0.2.

**5.** A process according to any one of the previous claims, characterized in that acetonitrile is used as the solvent.

**6.** A process according to any one of the previous claims, characterized in that the reaction is carried out at temperatures comprised between 0 ° C and 40 ° C.

**7.** A process according to any one of claims 1-5, characterized in that eseroline is reacted with heptylisocyanate which is prepared in situ starting from a 1-haloheptane and a metal cyanate in the presence of a quaternary ammonium salt, in polar aprotic solvents.

**8.** A process according to claim 1, wherein Meis potassium; $C_1$-$C_{12}$ alkyl is ethyl or n-butyl; X is chlorine or bromine.

**9.** A process according to claim 7, wherein the 1-haloheptane is 1-bromoheptane and the metal cyanate is potassium cyanate.

**10.** A process according to claim 7, wherein the polar aprotic solvent is acetonitrile.

European Patent Office

# EUROPEAN SEARCH REPORT

Application Number

EP 92 10 2199

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| D,A | EP-A-0 298 202 (MEDIOLANUM; CONSIGLIO NAZIONALE DELLE RICERCHE)<br>* page 3, line 51 - page 4, line 45 *<br>----- | 1 | C 07 D 487/04 //<br>A 61 K 31/40<br>(C 07 D 487/04<br>C 07 D 209:00<br>C 07 D 209:00 ) |
| | | | TECHNICAL FIELDS SEARCHED (Int. Cl.5) |
| | | | C 07 D |

The present search report has been drawn up for all claims

| Place of search | Date of completion of the search | Examiner |
|---|---|---|
| THE HAGUE | 07-05-1992 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)